# EUROPEAN PATENT APPLICATION

(11) **EP 1 543 824 A2**
(43) Date of publication of application: **22.06.2005**
(21) Application number: 04030027.9
(22) Date of filing: 17.12.2004
(51) Int. Cl.: A61K 7/48, A61K 31/426

(54) **Thiazole derivatives to counter advanced glycation**

(30) Priority: 17.12.2003 US 738412
(71) Applicant: Avon Products, Inc., Suffern NY 10901-5605 (US)
(72) Inventor: Hines, Michelle D., Lawnside, New Jersey 08045 (US); Jones, Brian C., Warwick, New York 10990 (US)
(74) Representative: VOSSIUS & PARTNER

(57) **Abstract**

Methods are disclosed for improving the appearance of skin by topical administration of thiazole derivatives which inhibit the formation of advanced glycation endproducts, break advanced glycation endproduct-associated crosslinks, and inhibit the function of glucose oxidase. Cosmetic and pharmaceutical compositions comprising the thiazole derivatives are also disclosed.

## Description

The present invention relates generally to compositions and methods for improving the texture and elasticity of skin. More specifically, the present invention relates to the use of thiazole derivatives to inhibit the formation of advanced glycation endproducts, break advanced glycation endproduct-associated crosslinks, and inhibit the function of glucose oxidase.

Deterioration of skin appearance and texture with age results in part from the accumulation of aberrant collagen and elastin proteins in the skin. These aberrant proteins possess impaired functionality due to the presence of advanced glycation endproducts ("AGEs"). AGEs are cross-linked protein structures that alter the chemical and mechanical properties of proteins such as collagen and elastin. The age related increase in AGEs is associated with the rigidity of the dermal fibers, leading to reduced skin tone, suppleness, and appearance.

AGEs are the ultimate chemical consequence of the non-enzymatic glycation reaction between reducing sugars and amine groups of proteins, amino acids, or lipids. In the skin, free amines present in proteins such as collagen and elastin react with the carbonyl moiety of glucose to form a covalent linkage known as a Shiff base. These early glycation products undergo a further series of chemical reactions known as "Maillard reactions," named after the French chemist who first discovered the process in 1912. The Maillard reactions ultimately result in crosslinked polymeric carbonyl-amine compounds. The crosslinked proteins characteristic of AGEs accumulate with age, particularly in long-lived proteins such as collagen and elastin. Thus, AGEs are an important molecular indication of cellular aging.

The formation of AGEs is further exacerbated by the presence of the enzyme glucose oxidase which catalyses the reaction of D-glucose to D-gluconolactone and hydrogen peroxide (H₂O₂). Cellular levels of H₂O₂ lead to direct protein damage as well as highly active oxygen species such as ketoaldeyhes produced by the oxidative degradation of glucose. Highly active oxygen species such as ketoaldehydes may undergo glycation in the present of protein, leading to AGE formation (Hunt 1991; Halliwell 1991).

In addition to the age-related deterioration of skin appearance and texture, AGEs have been implicated in the pathogenesis in a variety of diseases including diabetes (Brownlee 1995; Brownlee 1988), atherosclerosis (Brownlee 1986) Alzheimer's (Vitek 1994; Smith1994), uremia (Odani 1998), rheumatoid arthritis (Verzijl 2002), and smoking-associated damage (Cerami 1997). AGEs also play a role in the spoilage and browning of food (Spanyar 1976).

Prior approaches to ameliorating the effects of AGEs include employing inhibitors of AGE formation, such as aminoguanidine (Rahbar 1999; Kochakian 1996). Other approaches involve using crosslink "breakers" to disrupt the AGE-derived protein cross-links (Vasan 1996). This latter approach is promising due to the potential for reversing cellular damage that has already occurred. However, this approach has been of only limited value to date in cleaving AGE cross-links formed in skin collagen (Yang 2000).

Accordingly, there is a continuing need for compounds, compositions, and methods for inhibiting AGEs. There is also a continuing need for compounds, compositions, and methods for breaking crosslinks associated with AGEs. Further, there is a continuing need for compounds, compositions, and methods for inhibiting glucose oxidase.

It is therefore an object of the present invention to provide compounds, compositions, and methods, e.g. to improve the aesthetic appearance of skin, accomplishing one or more of the following objectives:
1. Inhibit the formation of AGEs;
2. Break or disrupt crosslinks associated with AGEs;
3. Inhibit glucose oxidase.

In accordance with the foregoing objectives, one aspect of the present invention provides compositions and methods for inhibiting the formation of advanced glycation endproducts. Another aspect of the invention provides compositions and methods for breaking crosslinks associated with advanced glycation endproducts. A further aspect of the invention provides compositions and methods for inhibiting glucose oxidase. In the preferred practice of the invention, the compounds, compositions, and methods disclosed achieve all three of these objectives. However, it will be understood that compounds, compositions, and methods that accomplish one or more of these objectives are considered to be within the scope of the present invention.

The compounds, compositions, and methods of the present invention are useful for preventing, treating, reversing, or ameliorating skin conditions associated with the presence of AGEs. However, the invention is not so limited. For example, it is contemplated that the compounds, compositions, and methods of the present invention will be useful for preventing, treating, reversing, or ameliorating any condition associated with the presence of AGEs.

It has surprisingly been found that thiazole derivatives of formula **I**, as shown below 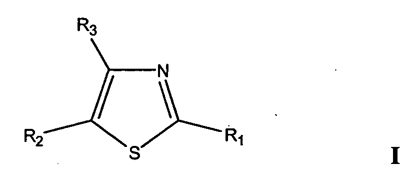 wherein R₁, R₂, and R₃ are independently selected from H; substituted or unsubstituted C₁-C₁₀ alkyl, alkenyl, alkynyl, aryl, cycloalkyl, and heterocyclic groups; NH₂; NRₐR_{b}; SH; SR; CN; halogen; OH; OR; SO₃⁻; SO₃R; NO₂; NO; C(=O)H; C(=O)R; C(=O)OH; C(=O)OR; C(=O)NRₐR_{b}; where R is selected from H, substituted or unsubstituted C₁-C₁₀ alkyl, alkenyl, alkynyl, aryl, cycloalkyl, and heterocyclic groups; and where Rₐ and R_{b} are independently selected from H, substituted or unsubstituted C₁-C₁₀ alkyl, alkenyl, alkynyl, aryl, cycloalkyl, and heterocyclic groups; or salts thereof, are useful for inhibiting the formation of AGEs or cleaving crosslinks associated with AGEs. Further, it has surprisingly been found that the thiazole derivatives of formula **I** are useful for inhibiting glucose oxidase.

Accordingly, one aspect of the invention provides compositions, such as cosmetic compositions, which are preferably suited for topical application, comprising an advanced glycation endproduct inhibiting or cleaving compound formula **I** or a salt thereof. Generally, the compound is present in an effective amount for treating or preventing advanced glycation enproduct related conditions when the composition is applied topically to the skin.

A second aspect of the present invention provides compositions, such as cosmetic compositions, which are preferably suited for topical application, comprising a glucose oxidase inhibiting compound of formula **I** or a salt thereof. Generally, the compound is present in an amount effective for inhibiting glucose oxidase when the composition is applied topically to the skin.

In the preferred practice of the above described embodiments of the invention, the compound according to formula **I** is 2-amino-4,5-dimethylthiazole or a salt thereof. Preferably, the salt is the hydrochloride salt of 2-amino-4,5-dimethylthiazole. Accordingly, another aspect of the invention provides compositions, such as cosmetic compositions, which are preferably suited for topical application, for preventing, treating, or reversing a condition associated with advanced glycation endproducts comprising 2-amino-4,5-dimethylthiazole or a salt thereof and a cosmetically acceptable vehicle, wherein the 2-amino-4,5-dimethylthiazole or salt thereof is present in an effective amount for treating, preventing, or reversing advanced glycation enproduct related conditions when applied topically to the skin.

In another aspect of the invention, methods are provided for preventing, treating, or reversing a condition associated with advanced glycation endproducts. The method according to this embodiment comprises administering an advanced glycation inhibiting or cleaving compound of formula **I** or a salt thereof.

In the preferred practice of this embodiment, the methods are employed to prevent, treat, or reverse AGE-associated skin conditions including, but not limited to, wrinkling, facial lines, dermatological signs of aging, loss of skin tone, loss of collagen, loss of elastin, loss of skin firmness, poor skin texture, loss of skin luster, loss of skin elasticity or resiliency, and thin skin.

Yet another aspect the invention provides methods for inhibiting the formation of advanced glycation endproducts comprising administering an advanced glycation endproduct inhibiting compound of formula **I** or a salt thereof.

As an additional aspect the invention provides methods for cleaving advanced glycation endproducts comprising administering an advanced glycation endproduct cleaving compound of formula **I** or a salt thereof.

Yet a further aspect of the invention provides methods for inhibiting glucose oxidase comprising administering a glucose oxidase inhibiting compound of the formula **I** or a salt thereof.

In the preferred practice of the foregoing embodiments, the methods comprise administering 2-amino-4,5-dimethylthiazole or a salt thereof.

Accordingly, a preferred embodiment according to the above described aspects of the present invention provides methods for preventing, treating, or reversing a condition associated with advanced glycation endproducts comprising administering 2-amino-4,5-dimethylthiazole or a salt thereof. Preferably the salt is the hydrochloride salt of 2-amino-4,5-dimethylthiazole. Preferred methods according to this embodiment are useful for treating AGE-associated skin conditions, including, but not limited to, wrinkling, facial lines, dermatological signs of aging, loss of skin tone, loss of collagen, loss of elastin, loss of skin luster, loss of skin firmness, poor skin texture, loss of skin elasticity or resiliency, and thin skin.

These and other aspects of the invention may be more clearly understood by reference to the following detailed description of the invention and the appended claims.

In the following description of the invention and the claims appended hereto, it is to be understood that the terms used have their ordinary and accustomed meanings in the art, unless otherwise specified. As used herein, the phrases "advanced glycation endproduct" and "AGE" are used synonymously and refer to any product resulting from the reaction of any sugar compound with any protein, lipid, or DNA molecule or any protein, lipid, or DNA containing molecule or structure. AGEs include but are not limited to those products of the "Maillard reaction" and those products of "post-Amadori rearrangements," as those terms are used in the art. The phrase "advanced glycation endproduct inhibition" embraces any process by which AGE formation is inhibited, including but not limited to inhibition of Shiff base formation, Amadori compound formation, and post-Amadori rearrangements. Further, the phrase "advanced glycation endproduct inhibition" embraces the inhibition of both reversible and irreversible reactions involved in the formation of AGEs.

As used herein, the phrases "advanced glycation endproduct inhibiting," "advanced glycation endproduct cleaving," "glucose oxidase inhibiting," and "advanced glycation endproduct inhibiting or cleaving," when used in reference to the compounds of formula **I**, are intended to exclude from the genus of compounds of the formula **I** those compounds that do not possess one or more of these properties. Accordingly, for example, "advanced glycation endproduct inhibiting or cleaving" compounds of formula I comprise those compounds of formula **I** that inhibit the formation of AGEs or cleave crosslinks associated with AGEs, or both.

The present invention provides thiazole derivative compounds of formula **I**, as shown below 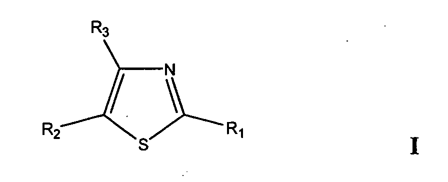 wherein R₁, R₂, and R₃ are independently selected from H; substituted or unsubstituted C₁-C₁₀ alkyl, alkenyl, alkynyl, aryl, cycloalkyl and heterocyclic groups; NH₂; NRₐR_{b}; SH; SR; CN; halogen; OH; OR; SO₃-; SO₃R; NO₂ NO; C(=O)H; C(=O)R; C(=O)OH; C(=O)OR; C(=O)NRₐR_{b}; where R is selected from H, substituted or unsubstituted C₁-C₁₀ alkyl, alkenyl, alkynyl, aryl, cycloalkyl and heterocyclic groups; and where Rₐ and R_{b} are independently selected from H, substituted or unsubstituted C₁-C₁₀ alkyl, alkenyl, alkynyl, aryl, cycloalkyl and heterocyclic groups; or salts thereof, that are useful for inhibiting the formation of AGEs or cleaving crosslinks associated with AGEs. Additionally, the compounds of formula I are useful for inhibiting glucose oxidase.

Exemplary optional substituents for R, R₁, R₂, R₃ and Rₐ und R_{b} are NH₂ and/or OH. Preferably, R, R₁, R₂, R₃ and Rₐ und R_{b} are unsubstituted. Exemplary aryl groups in the definition of R, R₁, R₂, R₃ and Rₐ und R_{b} have 6 to 18 or 6 to 12 carbon atoms. Exemplary cycloalkyl or heterocyclic groups have 3 to 8 ring members, such as 5, 6 or 7.

Preferred thiazole derivatives of formula **I** which inhibit the formation of AGEs and/or cleave crosslinks associated with AGEs and/or inhibit glucose oxidase are those wherein R₁, R₂, and R₃ are independently selected from H; substituted or unsubstituted C₁-C₄ alkyl, alkenyl, alkynyl groups; NH₂; SH; SR; CN; OH; or OR; where R is selected from substituted or unsubstituted C₁-C₄ alkyl, alkenyl, and alkynyl groups; and salts thereof.

In one preferred embodiment of the invention, the thiazole derivatives of formula **I** comprise at least one amino group (NH₂). The compounds according to this embodiment are preferably selected from those wherein R₁ is NH₂ and R₂ and R₃ are independently selected from the group consisting of methyl, ethyl, propyl, isopropyl, butyl, vinyl, allyl, methoxy, ethoxy, propoxy, and butoxy. In another preferred embodiment of the invention, R₃ is NH₂ and R₁ and R₃ are independently selected from the group consisting of methyl, ethyl, propyl, isopropyl, butyl, vinyl, allyl, methoxy, ethoxy, propoxy, and butoxy.

In another preferred embodiment of the invention, the thiazole derivatives of formula **I** comprise at least one methyl group (CH₃). More preferred thiazole derivatives are those wherein any two of R₁, R₂, and R₃ are methyl groups. The most preferred thiazole derivatives according to this embodiment are those wherein R₂ is a methyl group and either R₁ or R₃ is also a methyl group. One particularly useful compound according to this embodiment is the thiazole derivative of formula **I** wherein R₂ and R₃ are CH₃ and R₁ is NH₂. This compound is referred to herein as "2-amino-4,5-dimethyl-1,3-thiazole" or "2-amino-4,5-dimethylthiazole," in accordance with IUPAC nomenclature. Other suitable compounds which inhibit glycation and which may be given for illustration are 2-ethyl-4-methylthiazole and 2,4-dimethylthiazole.

It will be understood that any salt of the thiazole derivatives of formula **I** is within the scope of the invention. Illustrative salts may be, for example, chosen from salts with inorganic anions or cations such as halogen or nitrate salts or ammonia, alkali metal and alkaline earth metal salts, especially Na, NH₄⁺, F, Cl and Br. Salts with organic anions, such as acetates, may also be used. In a preferred embodiment, the thiazole derivatives of formula **I** are provided as hydrochloride salts. The hydrochloride salt of 2-amino-4,5-dimethylthiazole has also been found to be particularly useful in the practice of the invention.

The present invention provides compositions, such as cosmetic and pharmaceutical compositions, comprising one or more compound of formula **I.** For example, cosmetic compositions according to the invention may be used for preventing, treating, reversing, or ameliorating a condition associated with AGEs. Generally, they comprise a cosmetically acceptable vehicle. The compositions are typically applied topically to the skin. Pharmaceutical compositions according to the invention may be used, for example, for treating or preventing advanced glycolation endproduct related conditions or for inhibiting glucose oxidase. Generally, they comprise a pharmaceutically acceptable vehicle.

One further embodiment of the invention provides cosmetic or pharmaceutical compositions for topical application comprising an AGE inhibiting or cleaving compound formula **I** or a salt thereof. Generally, the compound is present in an effective amount for treating or preventing AGE related conditions when the composition is applied topically to the skin.

Another embodiment of the invention provides cosmetic or pharmaceutical compositions for topical application comprising a glucose oxidase inhibiting compound of formula **I** or a salt thereof. Generally, the compound is present in an amount effective for inhibiting glucose oxidase when the cosmetic composition is applied topically to the skin.

A further embodiment of the invention provides cosmetic or pharmaceutical compositions for topical application, for preventing, treating, reversing, or ameliorating a condition associated with AGEs comprising 2-amino-4,5-dimethylthiazole or a salt thereof and a cosmetically acceptable vehicle. Generally, the 2-amino-4,5-dimethylthiazole or salt thereof is present in an effective amount for treating, preventing, or reversing AGE related conditions when the cosmetic composition is applied topically to the skin.

The compositions according to the invention may comprise any amount of the thiazole derivatives of formula **I.** Preferably, the compositions comprise from about 0.01 to about 30 weight %, more preferably from about 0.5 to about 20 weight %, and most preferably from about 1 to about 5 weight % of the thiazole derivative, based on the total weight of the composition.

The compositions according to the present invention may be provided in one or more cosmetically or pharmaceutically acceptable vehicles. Cosmetically or pharmaceutically acceptable vehicles according to the invention include any vehicle known in the art, including, but not limited to, lotions, ointments, foams, mousses, masks, pomades, sprays, sticks, serums, towelettes, patches, solutions, creams, and gels.

The cosmetic or pharmaceutical compositions according to the invention may optionally comprise other active and inactive ingredients, including, but not limited to, excipients, fillers, emulsifying agents, antioxidants, surfactants, film formers, chelating agents, gelling agents, thickeners, emollients, humectants, moisturizers, vitamins, minerals, viscosity and/or rheology modifiers, sunscreens, keratolytics, depigmenting agents, retinoids, hormonal compounds, alpha-hydroxy acids, alpha-keto acids, anti-mycobacterial agents, antifungal agents, antimicrobials, antivirals, analgesics, lipidic compounds, anti-allergenic agents, H₁ or H₂ antihistamines, anti-inflammatory agents, anti-irritants, antineoplastics, immune system boosting agents, immune system suppressing agents, anti-acne agents, anesthetics, antiseptics, insect repellents, skin cooling compounds, skin protectants, skin penetration enhancers, exfollients, lubricants, fragrances, colorants, staining agents, depigmenting agents, hypopigmenting agents, preservatives, stabilizers, pharmaceutical agents, photostabilizing agents, and mixtures thereof.

The present invention also provides methods of using the compounds of formula **I.** In one embodiment of the invention, methods are provided for preventing, treating, reversing, or ameliorating a condition associated with AGEs comprising administering an AGE inhibiting or cleaving compound of formula **I** or a salt thereof.

Another embodiment of the invention is a method for inhibiting the formation of AGEs comprising administering an AGE inhibiting compound of formula **I** or a salt thereof.

Yet another embodiment of the invention is method for cleaving AGEs comprising administering an AGE cleaving compound of formula I or a salt thereof.

An additional embodiment of the invention is a method for inhibiting glucose oxidase comprising administering a glucose oxidase inhibiting compound of the formula **I** or a salt thereof.

In the preferred practice of the foregoing embodiments, the methods comprise administering 2-amino-4,5-dimethylthiazole or a salt thereof.

Accordingly, a preferred embodiment of the invention is a method for preventing, treating, or reversing a condition associated with AGEs comprising administering 2-amino-4,5-dimethylthiazole or a salt thereof. Preferably the salt is the hydrochloride salt of 2-amino-4,5-dimethylthiazole.

The methods may be employed for preventing, treating, reversing, or ameliorating a condition associated with AGEs in an organism. In the preferred practice of the invention, the methods are employed to prevent, treat, or reverse AGE-associated skin conditions including, but not limited to, wrinkling, facial lines, dermatological signs of aging, loss of skin tone, loss of collagen, loss of elastin, loss of skin firmness, poor skin texture, loss of skin luster, loss of skin elasticity or resiliency, and thin skin. As used herein, the phrase "improving the appearance of skin" refers to detectable improvement in any characteristic of skin, including but not limited to, the characteristics listed above.

In the preferred practice of the methods of the invention, the thiazole derivatives of formula **I** are applied topically to skin.

Other conditions for which the methods and the pharmaceutical compositions of the present invention are contemplated to be useful include, but are not limited to, diabetes, rheumatoid arthritis, Alzheimer's disease, uremia, neurotoxicity, and atherosclerosis.

However, the invention is not limited to AGE-associated conditions in living organisms. The methods disclosed are contemplated to be useful for preventing, treating, reversing, or ameliorating any AGE-associated condition including those which are not in association with a human or animal body, such as, for example, spoilage of food products.

Furthermore, in addition to topical administration, the compositions and methods of the invention may also be administered by, for example, oral or intravenous application and through nutritional supplements.

### EXAMPLE 1

This example describes the efficacy of a compound of the invention for inhibiting AGE formation.

A control tube, containing protein such as albumin, e.g. human or bovine serum albumin at a concentration of 400 mg/ml, and sugar, e.g. ribose at a concentration of 400 mM , forms a gel with little or no remaining liquid when incubated at 37°C for 5 to 7 days. Such a gel is indicative of extensive crosslinking associated with glycation. In contrast, a treatment tube, which contained all the contents of the control tube plus the AGE-inhibiting compound 2-amino-4,5-dimethylthiazole HCl, remained liquid, indicating that 2-amino-4,5-dimethylthiazole HCl is an effective AGE inhibitor.

### EXAMPLE 2

This example describes the efficacy of a compound of the invention for breaking crosslinks associated with AGEs using a sandwich enzyme-linked immunoabsorbent assay.

A mixture of protein, e.g. human or bovine serum albumin (50 mg/ml), and sugar, e.g. ribose (250 mM), was incubated to form cross-links representative of AGEs and was placed in multi-well plates. The plates were then treated with either 2-amino-4,5-dimethylthiazole HCl or buffer. After treatment, the plates were rinsed to remove any free protein liberated by cross-link breakage. An antibody reactive to the protein was then added to the plates to provide quantitative levels of protein within the plate. The ELISA protocol was as follows:
1. Mix equal volumes of human serum albumin (50 mg/ml) with ribose (250 m/M) with and without test article
2. add 100 µl of mixture to each well of a 96-well plate coated with collagen
3. incubate at 37°C for 5 days
4. wash wells 3 x with PBS + 0.5% Tween 20
5. add primary monoclonal antibody directed against human albumin
6. incubate at 37°C overnight
7. wash wells 3 x with PBS + 0.5% Tween 20
8. add secondary antibody directed against mouse IgG and conjugated to alkaline phosphatase
9. incubate at room temperature for 3 hours
10. add alkaline phosphate substrate
11. incubate at room temperature for 30 minutes
12. read absorbance at 405 nm using a spectrophotometer.

The plate treated with 2-amino-4,5-dimethylthiazole HCl had less antibody bound in the plate as compared to the control plate, indicating that 2-amino-4,5-dimethylthiazole HCl is effective in breaking cross-links associated with glycation.

### EXAMPLE 3

This example describes the effectiveness of a compound of the invention for inhibiting the enzyme glucose oxidase.

To test the efficacy of 2-amino-4,5-dimethylthiazole HCl for the inhibition of glucose oxidase, a commercially available glucose oxidase kit, such as provided by Molecular Probes, Eugene, Oregon Catalogue No. A-22189, was used. The assay indicated that the production of H₂O₂ was reduced by greater than 60% in a sample containing 2-amino-4,5-dimethylthiazole HCl as compared to a control sample, indicating that 2-amino-4,5-dimethylthiazole HCl is effective for the inhibition of glucose oxidase.

### EXAMPLE 4

Examples of compounds of formula I that are useful in the practice of the present invention include, but are not limited to, the compounds shown in Table 1.

**Table 1.**

| Compound | Source |
|---|---|
| 2-aminothiazole | Sigma-Aldrich #12,312-9 |
| 4,5-dimethylthiazole | C.A.S.* #3581-91-7 |
| 2-amino-4,5-dimethylthiazole hydrochloride | Sigma-Aldrich #17,440-8 |
| 2-amino-4,5-dimethylthiazole hydrobromide | Sigma-Aldrich #S45,752-3 |
| 2-amino-5-methylthiazole | Sigma-Aldrich #08652 |
| 2-amino-4-methylthiazole | Sigma-Aldrich #A6,600-6 |
| 2-amino-4-methylthiazole hydrochloride | Sigma-Aldrich #S36,653-6 |
| 2-amino-5-bromothiazole | C.A.S. #3034-22-8 |
| 2-amino-5-acetyl-4-methylthiazole hydrochloride | Sigma-Aldrich #S94,865-9 |
| ethyl 2-aminothiazole-4-acetate | Sigma-Aldrich #22,055-8 |
| 2-methyl-thiazole-4,5-diol | Sigma-Aldrich #S 13,764-2 |
| 2-amino-1,3-thiazole-5-sulfonic acid | Sigma-Aldrich #R66,309-3 |
| 2-amino-4-methyl-thiazole-5-sulfonic acid | Sigma-Aldrich #S13,971-8 |
| 2,4-diamino-5-phenylthiazol hydrobromide | C.A.S. #6020-54-8 |
| methyl 2-amino-4-methyl-1,3-thiazole-5-carboxylate | Sigma-Aldrich #R92,367-2 |
| ethyl 2-amino-1,3-thiazole-4-carboxylate | Sigma-Aldrich #R58,017-1 |
| 2-amino-4-methyl-thiazole-5-carboxylic acid dimethylamide | Sigma-Aldrich #R43,328-4 |
| 2,4-diamino-5-phenyl-thiazole, mono-hydrobromide | Sigma-Aldrich #R17,403-3 |
| 2-amino-4-methyl-thiazole-5-carboxylic acid ethyl ester | Sigma-Aldrich #R43,305-5 |
| ethyl 2-bromo-1,3-thiazole-5-carboxylate | Sigma-Aldrich #R57,977-7 |
| 2-amino-n-isopropyl-4-methyl-1,3-thiazole-5-carboxamide | Sigma-Aldrich #R95,028-9 |
| 2-acetamido-4-(trifluoromethyl)thiazole | Sigma-Aldrich #S88,344-1 |

| | |
|---|---|
| *Chemical Abstracts Registry Number. | |

The invention having been described by the foregoing description of the preferred embodiments, it will be understood that the skilled artisan may make modifications and variations of these embodiments without departing from the spirit or scope of the invention as set forth in the following claims.

### References:

1. Brownlee M. *Advanced protein glycosylation in diabetes and aging.* Ann. Rev. Med. 1995; 46:223-34.
2. Brownlee M, Cerami A, Vlassara H.A. *Advanced glycosylation endproducts in tissue and the biochemical basis of diabetic complications.* N. Engl. J. Med., 1988, 318, 1315-1321.
3. Brownlee M, Vlassara H, Kooney A, Ulrich P, Cerami A. *Aminoguanidine prevents diabetes-induced arterial wall protein cross-linking.* Science. 1986; 232: 1629-1632.
4. Vitek MP, Bhattacharya K, Glendening JM, Stopa E, Vlassara H, Bucala R, et al. *Advanced glycation end products contribute to amyloidosis in Alzheimer disease.* Proc. Natl. Acad. Sci. USA 1994; 91:4766-70.
5. Smith,M.A. et al. 1994. *Advanced Maillard reaction end products are associated with Alzheimer disease pathology.* Proc. Natl. Acad. Sci.USA 91:5710-5714.
6. Vitek MP, Bhattacharya K, Glendening JM, Stopa E, Vlassara H, Bucala R, et al. *Advanced glycation end products contribute to amyloidosis in Alzheimer disease.* Proc. Natl. Acad. Sci. USA 1994;91:4766-70.
7. Smith,M.A. et al. *1994. Advanced Maillard reaction end products are associated with Alzheimer disease pathology.* Proc. Natl. Acad. Sci.USA 91:5710-5714.
8. Verzijl N. et al., *Crosslinking by advanced glycation end product increases the stiffness of the collagen network in human articular cartilage: A possible mechanism through which age is a risk factor for osteoarthritis.* Arthritis Rheum. 2002. 46(1): 114-123.
9. Odani,H., Shinzato,T., Usami,J., Matsumoto,K., Brinkmann Frye,E., Baynes,J.W., and Maeda,K. 1998. *Imidazolium crosslinks derived from reaction of lysine with glyoxal and methylglyoxal are increased in serum proteins of uremic patients: evidence for increased oxidative stress in uremia.* FEBS Lett. 427:381-385.
10. Cerami,C., Hounds,H., Nicholl,I., Mitsuhashi,T., Giordano,D., Vanpatten,S., Lee,A., Al-Abed,Y., Vlassara,H., Bucala,R. et al. 1997. *Tobacco smoke is a source of toxic reactive glycation products.* Proc. Natl. Acad. Sci.USA 94:13915-13920.
11. Spanyar, P., *The Importance of the Maillard Reaction in Food Chemistry,* Period. Polytech. Chem. Eng., 20 (1976), pp. 371-378.
12. Rahbar, S., et al., I. *Novel inhibitors of advanced glycation endproducts.* Biochem. Biophys. Res. Commun., 262:651-656, 1999.
13. Kochakian, M. et al. *Chronic dosing with aminoguanidine and novel advanced glycosylation end product-formation inhibitors ameliorates cross-linking of tail tendon collagen in STZ-induced diabetic rats.* Diabetes 45:1694-1700, 1996.
14. Vasan, S. et al. *An agent cleaving glucose-derived protein crosslinks in vitro and in vivo.* Nature, 382:275-278, 1996.
15. Yang, S., Thorpe, S. R., and Baynes, J. W. *AGE-breakers fail to break cross-links in skin collagen of diabetic rats.* Diabetes, 49 (Suppl. 1), A130, 2000.
16. Hunt, J. V., and Wolff, S. (1991) *Oxidative glycation and free radical production: a causal mechanism of diabetic complications.* Free Radical Res. Commun.**12**, 115-123.
17. Halliwell, B., and Gutteridge, J. M. C. (1991) *Free Radicals in Biology and Medicine,* 296 pp., Clarendon Press; Oxford.

## Claims

1. A cosmetic or pharmaceutical composition for topical application comprising an advanced glycation endproduct inhibiting or cleaving compound or glucose oxidase inhibiting compound of the formula 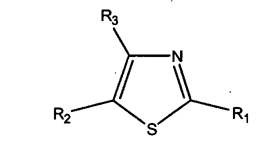 wherein R₁, R₂, and R₃ are independently selected from H; substituted or unsubstituted C₁-C₁₀ alkyl, alkenyl, alkynyl, aryl, cycloalkyl, and heterocyclic groups; NH₂; NRₐR_{b}; SH; SR; CN; halogen; OH; OR; SO₃⁻; SO₃R; NO₂; NO; C(=O)H; C(=O)R; C(=O)OH; C(=O)OR; C(=O)NRₐR_{b}; where R is selected from H, substituted or unsubstituted C₁-C₁₀ alkyl, alkenyl, alkynyl, aryl, cycloalkyl, and heterocyclic groups; and where Rₐ and R_{b} are independently selected from H, substituted or unsubstituted C₁-C₁₀ alkyl, alkenyl, alkynyl, aryl, cycloalkyl, and heterocyclic groups;
or a salt thereof;
and a cosmetically or pharmaceutically acceptable vehicle.

2. A composition comprising a glucose oxidase inhibiting compound or an advanced glycation endproduct inhibiting or cleaving compound of the formula 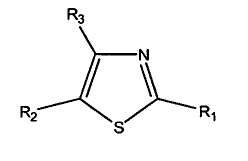 wherein R₁, R₂, and R₃ are independently selected from H; substituted or unsubstituted C₁-C₁₀ alkyl, alkenyl, alkynyl, aryl, cycloalkyl, and heterocyclic groups; NH₂; NRₐR_{b}; SH; SR; CN; halogen; OH; OR; SO₃⁻; SO₃R; NO₂; NO; C(=O)H; C(=O)R; C(=O)OH; C(=O)OR; C(=O)NRₐR_{b}; where R is selected from H, substituted or unsubstituted C₁-C₁₀ alkyl, alkenyl, alkynyl, aryl, cycloalkyl, and heterocyclic groups; and where Rₐ and R_{b} are independently selected from H, substituted or unsubstituted C₁-C₁₀ alkyl, alkenyl, alkynyl, aryl, cycloalkyl, and heterocyclic groups;
or a salt thereof;
wherein said compound is present in an amount effective for inhibiting glucose oxidase or for treating or preventing advanced glycation end product related conditions.

3. The composition of claim 2, which is a cosmetic or pharmaceutical composition further comprising a cosmetically or pharmaceutically acceptable vehicle.

4. The composition of any of claims 2 or 3, which is adapted for topical application.

5. The composition of any of claims 1 to 4, wherein R₁, R₂, and R₃ are independently selected from H; substituted or unsubstituted C₁-C₄ alkyl, alkenyl, alkynyl groups; NH₂; SH; SR; CN; OH; or OR; where R is selected from substituted or unsubstituted C₁-C₄ alkyl, alkenyl, and alkynyl groups.

6. The composition of any of claims 1 to 5, wherein R₁ is NH₂ and R₂ and R₃ are independently selected from the group consisting of methyl, ethyl, propyl, isopropyl, butyl, vinyl, allyl, methoxy, ethoxy, propoxy, and butoxy.

7. The composition of any of claims 1 to 5, wherein R₃ is NH₂ and R₁ and R₃ are independently selected from the group consisting of methyl, ethyl, propyl, isopropyl, butyl, vinyl, allyl, methoxy, ethoxy, propoxy, and butoxy.

8. The composition of claim 6 or 7, wherein R₂ is CH₃.

9. The composition of claim 7, wherein R₃ is CH₃.

10. The composition of claim 5, wherein R₁ is NH₂.

11. The composition of claim 5, wherein R₃ is NH₂.

12. The composition of any of claims 1 to 11, wherein said compound comprises from about 0.01 to about 30 weight % of the total composition.

13. The composition of any of claims 1 to 4 or 12, wherein said compound is 2-amino-4,5-dimethylthiazole, or a salt thereof.

14. The composition of claim 13, wherein said compound is the hydrochloride salt of 2-amino-4,5-dimethylthiazole.

15. The composition of any of claims 1 to 14, wherein said compound is present in an amount effective for treating or preventing advanced glycation end product related skin conditions.

16. The composition of claim 15, wherein said compound is present in an amount effective to inhibit or cleave advanced glycation end products in the skin.

17. The composition of any of claims 1 to 14, wherein said compound is present in an amount effective to inhibit glucose oxidase in the skin.

18. Use of a cosmetic composition according to any of claims 1 and 3 to 17 for preventing, treating, ameliorating or reversing a condition associated with advanced glycation endproducts.

19. The use of claim 18, wherein said condition is selected from the group consisting of: wrinkling, facial lines, dermatological signs of aging, loss of skin tone, loss of collagen, loss of elastin, loss of skin firmness, poor skin texture, loss of skin luster, loss of skin elasticity or resiliency, and thin skin.

20. A method for improving the appearance of skin, said method comprising topically applying to the skin a composition according to any of claims 1 to 17.

21. The method of claim 20, wherein said improvement is to a condition selected from the group consisting of: wrinkling, facial lines, dermatological signs of aging, loss of skin tone, loss of collagen, loss of elastin, loss of skin firmness, poor skin texture, loss of skin luster, loss of skin elasticity or resiliency, and thin skin.

22. Use of an advanced glycation endproduct inhibiting or cleaving compound or glucose oxidase inhibiting compound of the formula 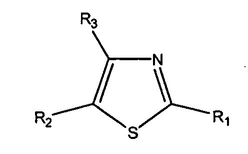 wherein R₁, R₂, and R₃ are independently selected from H; substituted or unsubstituted C₁-C₁₀ alkyl, alkenyl, alkynyl, aryl, cycloalkyl, and heterocyclic groups; NH₂; NRₐR_{b}; SH; SR; CN; halogen; OH; OR; SO₃⁻; SO₃R; NO₂; NO; C(=O)H; C(=O)R; C(=O)OH; C(=O)OR; C(=O)NRₐR_{b}; where R is selected from H, substituted or unsubstituted C₁-C₁₀ alkyl, alkenyl, alkynyl, aryl, cycloalkyl, and heterocyclic groups; and where Rₐ and R_{b} are independently selected from H, substituted or unsubstituted C₁-C₁₀ alkyl, alkenyl, alkynyl, aryl, cycloalkyl, and heterocyclic groups;
or a salt thereof for the preparation of a medicament for treating or preventing advanced glycation endproduct related conditions or for inhibiting glucose oxidase.

23. The use of claim 22, wherein R₁, R₂, and R₃ are independently selected from H; substituted or unsubstituted C₁-C₄ alkyl, alkenyl, alkynyl groups; NH₂; SH; SR; CN; OH; or OR; where R is selected from substituted or unsubstituted C₁-C₄ alkyl, alkenyl, and alkynyl groups.

24. The use of claim 23, wherein R₂ and R₃ are CH₃ and R₁ is NH₂.

25. The use of any of claims 22 to 24 , wherein said compound comprises from about 0.01 to about 30 weight % of the total composition.

26. The use of claim 25, wherein said compound comprises from about 1 to about 5 weight % of the total composition.

27. The use of any of claims 22, 25 or 26 , wherein said compound is 2-amino-4,5-dimethylthiazole or a salt thereof.

28. The use of claim 27, wherein said compound is the hydrochloride salt of 2-amino-4,5-dimethylthiazole.

29. The use of any of claims 22 to 28, wherein said condition is selected from the group consisting of diabetes, rheumatoid arthritis, Alzheimer's disease, uremia, neurotoxicity, and atherosclerosis.

30. A cosmetic method for treating or preventing advanced glycation endproduct related conditions or for inhibiting glucose oxidase comprising administering a composition according to any of claims 1 to 17.

31. A method for treating or preventing advanced glycation endproduct related conditions or for inhibiting glucose oxidase not in association with a human or animal body, comprising applying a composition according to any of claims 2 or 5 to 14.

32. The method according to claim 31, wherein the advanced glycation endproduct related condition is the spoilage of food products.
